# EUROPEAN PATENT APPLICATION

(11) **EP 4 410 825 A1**
(43) Date of publication of application: **07.08.2024**
(21) Application number: 23382104.0
(22) Date of filing: 03.02.2023
(51) Int. Cl.: C07K 14/47, A61K 31/728, A61K 38/17, A61K 47/69, A61K 48/00, A61K 49/00, A61P 35/00, B82Y 5/00, C12N 15/88

(54) **FRAGMENTS OF THE N-TERMINAL DOMAIN OF GSDMB FOR THE TREATMENT OF CANCER**

(71) Applicant: Servizo Galego de Saude, 15703 Santiago de Compostela (ES); Fundación Instituto de Investigación Sanitaria de Santiago de Compostela (FIDIS), Santiago de Compostela (ES); Consorcio Centro de Investigacion Biomedica en Red, 28029 Madrid (ES); Universidad Autónoma de Madrid, 28049 Madrid (ES); Universidade De Santiago De Compostela, 15782 Santiago de Compostela (ES)
(72) Inventor: DE LA FUENTE FREIRE, María, 15706 Santiago de Compostela (ES); LORES TOURIÑO, Saínza, 15706 Santiago de Compostela (ES); SARRIÓ LÓPEZ, José David, 28049 Madrid (ES); GAMEZ CHIACHIO, Manuel, 28029 Madrid (ES); MORENO BUENO, Gema, 28029 Madrid (ES)
(74) Representative: Hoffmann Eitle

(57) **Abstract**

The present invention generally refers to an isolated polypeptide comprising a fragment of the N-Terminal domain of GSDMB, wherein said fragment is characterized by comprising amino acid sequence 1-233 of the N-Terminal domain of GSDM-B or a functional equivalent thereof or a polynucleotide sequence coding for the said polypeptide, for use in the treatment of cancer.

## Description

### TECHNICAL FIELD

The invention relates to the field of cancer and, more particularly, to polypeptides, compositions and their use in medicine, particularly in the prevention and/or treatment of cancer.

### BACKGROUND ART

After its discovery in 2000, Gasdermin (GSDM) protein family, composed of 6 genes in humans and 10 in mice [1], has become increasingly popular. However, it was not until 2015, when GSDM-D was described as the executor of pyroptosis [1-3], currently named GSDM-mediated programmed cell death. Since then, great efforts have been made to understand its mechanism and develop innovative treatments against cancer cells [1-5]. All GSDMs, except PJVK, share the interconnected and mainly conserved at structural level, C-terminal (CT) and N-terminal (NT) domains. However, NT domain uniquely can interact with specific lipids and be inserted into the cell membrane, with a consequent pore formation and cell death. In contrast, the interaction between CT and NT inhibits this binding and consequent cell damage. Thus, the cleavage of this interdomain region as well as the NT delivery into the cancer cells lead to pyroptotic activation [6-9]. Additional inflammatory cell death processes were also evidenced to be carried out by GSDMs, such as NETosis in the case of GSDM-D, or "necrosis secondary to apoptosis in the case of GSDM-E, as well as mitochondrial damage [1,10,11].

Particularly, pyroptosis was pointed out as a combination of pyro and ptosis with the meaning of fire and falling, respectively [12]. It is a type of lytic cell death that is gaining more and more relevance in the field of cancer [14-16]. Pyroptosis and apoptosis shared some characteristics, such as DNA damage and chromatin condensation [13] but pyroptosis have also specific features. In this regard, pyroptotic morphological changes are characterized by multiple bubble-like protrusions with rupture of the cell membrane, and finally, the release of specific cytokines promoting systemic inflammatory effects [17].

Pyroptosis has emerged as an innovative therapeutic alternative, especially to apoptosis-resistance acquired, one of the main problems after chemotherapy treatment.

### DESCRIPTION OF EMBODIMENTS

In this invention, we follow a gene therapy strategy to mediate the delivery of a NT fragment to breast cancer cells. Due to its apparently key role on breast cancer [19,21], we focused on GSDM-B, and designed a plasmid DNA (pDNA) encoding for the NT domain. For a successful gene delivery, we decided to use putrescine nanosystems (PSN), previously developed and showing high ability to associate nucleic acids and achieve its delivery in triple negative breast cancer cells *in vitro* and *in vivo.* Additionally, we added a coating of a natural-occurring polysaccharide hyaluronic acid (HA) to maximise the interaction of the plasmid-loaded nanosystems with cancer cells [22-25]. Its use has exponentially increased since the 90's and proved a preferential tumour accumulation and improved cellular uptake [26]. Finally, we performed *in vitro* and *in vivo* experiments, in relevant models of TNBC, to prove the potential of our approach. As shown in the examples, a fast cellular internalization was achieved in triple negative breast cancer cell line, MDA-MB-231. In addition, a great therapeutic activity was exhibited in the same cellular model. Remarkably, this invention reports for the first time, the treatment of cancer animal models with the N-terminal domain of GSDMB.

Therefore, a first aspect of the invention refers to an isolated polypeptide fragment of the N-Terminal domain of GSDMB (from hereinafter polypeptide of the invention), wherein said fragment is characterized by comprising, consisting essentially of or consisting of amino acid sequence 1-233 of the N-Terminal domain of GSDMB (SEQ ID NO 1), or a functionally equivalent variant of said polypeptide fragment, or a polynucleotide sequence coding for the said polypeptide fragment.

Therefore, the polypeptide fragment comprises, consists of or consists essentially of SEQ ID NO: 1 as shown below (amino acid sequence 1-233 of the N-Terminal domain of GSDMB):

MFSVFEEITRIVVKEMDAGGDMIAVRSLVDADRFRCFHLVGEKRTFFGCRHYTTGLTLMDILDTDGDKW
LDELDSGLQGQKAEFQILDNVDSTGELIVRLPKEITISGSFQGFHHQKIKISENRISQQYLATLENRKLKRELP
FSFRSINTRENLYLVTETLETVKEETLKSDRQYKFWSQISQGHLSYKHKGQREVTIPPNRVLSYRVKQLVFP
NKETMNIHFRGKTKSFPE

In this context, "consisting essentially of" means that the specified molecule would not contain any additional sequences that would alter the activity of SEQ ID NO: 1.

Thus, the invention refers to a polypeptide fragment comprising, consisting of, or consisting essentially of SEQ ID NO: 1. The polypeptide that can be used in the present invention may derive from any mammal species, including but not being limited to domestic and farm animals (cows, horses, pigs, sheep, goats, dog, cats or rodents), primates and humans.

In a preferred embodiment, the invention relates to a polypeptide fragment consisting of SEQ ID NO: 1 or a functionally equivalent variant of said polypeptide.

In a preferred embodiment, the polypeptide of the invention comprises, consists of or consists essentially of the sequence as shown in SEQ ID NO: 2 (amino acid sequence 1-242 of the N-Terminal domain of GSDMB):

MFSVFEEITRIVVKEMDAGGDMIAVRSLVDADRFRCFHLVGEKRTFFGCRHYTTGLTLMDILDTDGDKW
LDELDSGLQGQKAEFQILDNVDSTGELIVRLPKEITISGSFQGFHHQKIKISENRISQQYLATLENRKLKRELP
FSFRSINTRENLYLVTETLETVKEETLKSDRQYKFWSQISQGHLSYKHKGQREVTIPPNRVLSYRVKQLVFP
NKETMNIHFRGKTKSFPEEKDGASSCL

In a preferred embodiment, the invention relates to a polypeptide consisting of SEQ ID NO: 2 or a functionally equivalent variant of said polypeptide.

In a preferred embodiment, the polypeptide of the invention comprises, consists of or consists essentially of the sequence shown in SEQ ID NO: 3 (amino acid sequence 1-233 of the N-Terminal domain of GSDMB):

MFSVFEEITRIVVKEMDAGGDMIAVRSLVDADRFRCFHLVGEKRTFFGCRHYTTGLTLMDILDTDGDKW
LDELDSGLQGQKAEFQILDNVDSTGELIVRLPKEITISGSFQGFHHQKIKISENRISQQYLATLENRKLKRELP
FSFRSINTRENLYLVTETLETVKEETLKSDRQYKFWSQISQGHLSYKHKGQREVTIPPNRVLSYRVKQLVFP
NKETMNIHFRGKTKSFPEEKDGASSCLGKSLGSEDSRNMKEKLEDMESVLKDLTEEKRKD

In a preferred embodiment, the invention relates to a polypeptide consisting of the polypeptide of SEQ ID NO: 3 or a functionally equivalent variant of said polypeptide.

The term "functionally equivalent variant", when referring to any of SEQ ID NO: 1 to 3 refers to any polypeptide which substantially preserves the tumor suppressor activity of any of these sequences. In particular, suitable functional variants of any of SEQ ID NO: 1 to 3 are those showing a degree of identity with respect to any one of these peptides of about greater than 25% amino acid sequence identity, such as 25%, 30%, 40%, 50%, 60%, 65%, 70%, 75%, 80%, 85%, 90%, 91%, 92%, 93%, 94%, 95%, 96%, 97%, 98% or 99%. The degree of identity between two polypeptides is determined using computer algorithms and methods that are widely known for the persons skilled in the art. The identity between two amino acid sequences is preferably determined by using the BLASTP algorithm as described previously [BLAST Manual, Altschul, S., et al., NCBI NLM NIH Bethesda, Md. 20894, Altschul, S., et al., J. Mol. Biol. 1990;215: 403-410]. In a preferred embodiment, the sequence identity is determined throughout the whole length of the polypeptide of SEQ ID NO: 1 to 3.

The functionally equivalent variants of the polypeptide of the invention may also include post-translational modifications, such as glycosylation, acetylation, isoprenylation, myristoylation, proteolytic processing, etc.

Alternatively, suitable functional variants of the targeting peptide are those wherein one or more positions within the polypeptide of SEQ ID NO: 1 to 3 contain an amino acid which is a conservative substitution of the amino acid present in the protein mentioned above. "Conservative amino acid substitutions" result from replacing one amino acid with another having similar structural and/or chemical properties. For example, the following six groups each contain amino acids that are conservative substitutions for one another: 1) Alanine (A), Serine (S), Threonine (T); 2) Aspartic acid (D), Glutamic acid (E); 3) Asparagine (N), Glutamine (Q); 4) Arginine (R), Lysine (K); 5) Isoleucine (I), Leucine (L), Methionine (M), Valine (V); and 6) Phenylalanine (F), Tyrosine (Y), Tryptophan (W). Selection of such conservative amino acid substitutions is within the skill of one of ordinary skill in the art and is described, for example, by Dordo et al., (J. Mol. Biol, 1999, 217;721-739) and Taylor et al., (J. Theor. Biol., 1986, 119:205-218).

In a preferred embodiment, a polypeptide is considered a functionally equivalent variant of any of SEQ ID NO: 1 to 3 if it shows a tumor suppressor activity in one or more assays which is at least 10%, 20%, 30%, 40%, 50%, 60%, 70%, 80%, 90% or 100% of the activity of any of SEQ ID NO: 1 to 3.

From hereinafter the polypeptide or functionally equivalent variant of the polypeptide according to the invention refers to a polypeptide comprising, consisting of or consisting essentially of any one of sequences SEQ ID NO: 1 to 3 or any functionally equivalent thereof.

In a preferred embodiment, the polypeptide or functionally equivalent variant of the polypeptide according to the invention has less than 300 amino acids in length, preferably 275 amino acids in length or less, more preferably between 233 and 250 amino acids in length, more preferably between 233 and 240 amino acids in length, more preferably between 233 and 235 amino acids in length, still more preferably about 233 amino acids in length.

In another aspect, the invention relates to a conjugate comprising:
a) the polypeptide or functionally equivalent variant of said polypeptide according to the invention and
b) a chemical moiety that facilitates cellular uptake of the polypeptide or of the functionally equivalent variant of said polypeptide.

The term "conjugate", as used herein, refers to two or more compounds which are covalently linked together so that the function of each compound is retained in the conjugate.

In preferred embodiments, the conjugates according to the invention comprise at least 1, at least 2, at least 3, at least 4, at least 5, at least 6, at least 7, at least 8, at least 9, at least 10 or more chemical moieties that facilitate cellular uptake of the polypeptide or of the functionally equivalent variant of said polypeptide.

In one embodiment, the chemical moiety that facilitates cellular uptake of the polypeptide is a lipid or a fatty acid. A fatty acid generally is a molecule comprising a carbon chain with an acidic moiety (e.g., carboxylic acid) at an end of the chain. The carbon chain of a fatty acid may be of any length, however, it is preferred that the length of the carbon chain be of at least 2, 3, 4, 5, 6, 7, 8, 9, 10, 11, 12, 13, 14, 15, 16, 17, 18, 19, 20 or more carbon atoms, and any range derivable therein. In certain embodiments, the length of the carbon chain is from 4 to 18 carbon atoms in the chain portion of the fatty acid. In certain embodiments the fatty acid carbon chain may comprise an odd number of carbon atoms, however, an even number of carbon atoms in the chain may be preferred in certain embodiments. A fatty acid comprising only single bonds in its carbon chain is called saturated, while a fatty acid comprising at least one double bond in its chain is called unsaturated. The fatty acid may be branched, though in preferable embodiments of the present invention, it is unbranched. Specific fatty acids include, but are not limited to, linoleic acid, oleic acid, palmitic acid, linolenic acid, stearic acid, lauric acid, myristic acid, arachidic acid, palmitoleic acid, arachidonic acid.

In a preferred embodiment, the chemical moiety that facilitates cellular uptake of the polypeptide is a cell penetrating peptide sequence, in which case, the conjugate is a fusion protein comprising a polypeptide of the invention or the functionally equivalent variant of said polypeptide and the cell penetrating peptide sequence.

The term "cell penetrating peptide sequence" is used in the present specification interchangeably with "CPP", "protein transducing domain" or "PTD". It refers to a peptide chain of variable length that directs the transport of a protein inside a cell. The delivering process into cell commonly occurs by endocytosis but the peptide can also be internalized into cell by means of direct membrane translocation. CPPs typically have an amino acid composition that either contains a high relative abundance of positively charged amino acids such as lysine or arginine or has sequences that contain an alternating pattern of polar/charged amino acid and non-polar, hydrophobic amino acids.

In another aspect, the invention relates to a polynucleotide encoding a polypeptide according to the invention or a conjugate according to the invention (preferably a polypeptide as exemplified in any of sequences SEQ ID NO: 1 to 3).

The terms "polynucleotide", "nucleic acid" and "nucleic acid molecule" are used interchangeably to refer to polymeric forms of nucleotides of any length. The polynucleotides may contain deoxyribonucleotides, ribonucleotides, and/or their analogs. Nucleotides may have any three-dimensional structure, and may perform any function, known or unknown. The term "polynucleotide" includes, for example, single-stranded, double-stranded and triple helical molecules, a gene or gene fragment, exons, introns, mRNA, tRNA, rRNA, ribozymes, cDNA, recombinant polynucleotides, branched polynucleotides, plasmids, vectors, isolated DNA of any sequence, isolated RNA of any sequence, nucleic acid probes, and primers. In addition to a native nucleic acid molecule, a nucleic acid molecule of the present invention may also comprise modified nucleic acid molecules. As used herein, mRNA refers to an RNA that can be translated in a cell.

Preferably, the polynucleotide is a DNA sequence, such as a cDNA sequence, or a polyribonucleotide sequence comprising a sequence from a stable mRNA or an mRNA, wherein the DNA sequence or the polyribonucleotide sequence that can be efficiently translated to the polypeptide according to the invention or a conjugate according to the invention.

In another aspect, the invention relates to a vector comprising a polynucleotide of the invention.

The term "vector", as used herein, refers to a nucleic acid sequence comprising the necessary sequences so that after transcribing and translating said sequences in a cell a polypeptide encoded by the polynucleotide of the invention is generated. Said sequence is operably linked to additional segments that provide for its autonomous replication in a host cell of interest. Preferably, the vector is an expression vector, which is defined as a vector which, in addition to the regions of the autonomous replication in a host cell, contains regions operably linked to the nucleic acid of the invention and which are capable of enhancing the expression of the products of the nucleic acid according to the invention. The vectors of the invention can be obtained by means of techniques widely known in the art.

Examples of vectors include, but are not limited to, viral vectors, naked DNA or RNA expression vectors, plasmid, cosmid or phage vectors, DNA or RNA expression vectors associated with cationic condensing agents, DNA or RNA expression vectors encapsulated in liposomes, and certain eukaryotic cells, such as producer cells. In a preferred embodiment, the vector according to the invention is use according to the invention by using a delivery system or by being encapsulated with a nano or micro-emulsion or system, an exosome, a lipid nanoparticle, a polymeric nanoparticle, a natural or artificial lipoprotein particle, a cationic lipid, a protein-nucleic acid complex, a liposome, a virosome, or a polymer.

In an even more preferred embodiment, the delivery system or encapsulation system according to the invention is a nano or micro-emulsion system comprising vitamin E (vit E) and sphingomyelin (SM) and a cationic or ionizable lipid, preferably a cationic or ionizable lipid comprising or consisting of putrescine. Preferably, said carrier or encapsulation system is formulated or obtained by an alcohol, such as ethanol, injection method, wherein vitamin E (VitE), sphingomyelin (SM) and the cationic or ionizable lipid, preferably a cationic or ionizable lipid comprising putrescine, more preferably putrescine modified with oleamide (Pt), are dissolved in the alcohol and injected in an aqueous medium, with or without additional mixing by homogenization, and wherein the polynucleotide according to the invention or the vector according to the invention, preferably a plasmid comprising the necessary sequences so that after transcribing and translating said sequences in a cell a polypeptide encoded by the polynucleotide of the invention is generated, is associated to the nanocarriers of the nano or microsystem. Preferably, the said nano or microsystem is further characterized by being coated with hyaluronic acid or a salt thereof, such as sodium hyaluronate.

Preferably, the nano or micro-emulsion system comprising vitamin E (vit E) and sphingomyelin (SM) and a cationic or ionizable lipid according to the invention, is produced by using vitamin E (VitE) and sphingomyelin (SM) at ratios (vitamin E (VitE) : sphingomyelin (SM)) of from 1:1 to 500:1, preferably from 1:1 to 100:1, preferably from 1:1 to 20:1, preferably from 5:1 to 15:1, more preferably about 10:1. The amount of the cationic or ionizable lipid is usually in a ratio (SM: cationic or ionizable lipid) of from 1:1 to 5:1, preferably about 2:1.

A non-limiting example of preparing such system is by mixing 5 mg of vitamin E (VitE), 0.5 mg of sphingomyelin (SM) and 0.25 mg of putrescine modified with oleamide (Pt) dissolved in 100 µl of ethanol and injected in 1 ml of Molecular Grade Water. Nanosystems are instantaneous formed. Then, 5 µg of pDNA can be dissolved in 100 µL of H₂O nuclease-free and added over 100 µL of preformed nanocarriers, for 20 min under stirring to achieve the association. Next, 20µL of sodium hyaluronate at 1 mg/mL can be optionally added to the nanosystems, and those were stirred for 5 min to achieve the coating.

The person skilled in the art can find more information on how to prepare these systems made of vitamin E (vit E) and sphingomyelin (SM) and a cationic or ionizable lipid in WO2019/138139.

The vector of the invention, preferably encapsulated in the delivery system or encapsulation system according to the invention, may be used to transform, transfect or infect cells that can be transformed, transfected or infected by said vector. Said cells may be prokaryotic or eukaryotic. Such transformation, transfection or infection of cells may be performed "in vivo" such as in a therapeutic method or "in vitro".

The vector preferably comprises the polynucleotide of the invention operationally bound to sequences that regulate the expression of the polynucleotide of the invention. The regulatory sequences of use in the present invention may be nuclear promoters or, alternatively, enhancer sequences and/or other regulatory sequences that increase expression of the heterologous nucleic acid sequence. In principle, any promoter can be used in the present invention provided said promoter is compatible with the cells wherein the polynucleotide is to be expressed. Thus, promoters suitable for realizing the present invention include, but are not necessarily limited to, constitutive promoters such as derivatives of eukaryotic virus genomes such as polyoma virus, adenovirus, SV40, CMV, avian sarcoma virus, hepatitis B virus, the metallothionein gene promoter, the herpes simplex virus thymidine kinase gene promoter, LTR regions of retroviruses, the immunoglobulin gene promoter, the actin gene promoter, the EF-1alpha gene promoter as well as inducible promoters wherein protein expression depends on the addition of a molecule or exogenous signal, such as tetracycline systems, the NFκB/UV light system, the Cre/Lox system and the heat shock genes promoter, the regulable RNA polymerase II promoters described in WO/2006/135436 and tissue-specific promoters.

In another aspect, the invention relates to a pharmaceutical composition comprising a pharmaceutically effective amount of a polypeptide or functional equivalent variant of said polypeptide according to the invention, a conjugate according to the invention, a polynucleotide according to the invention, or a vector according to the invention, preferably carried or encapsulated by a delivery system or encapsulation system according to the invention, and optionally a pharmaceutically acceptable excipient.

As it is used in the present invention, the expression "pharmaceutical composition" relates to a formulation that has been adapted for administering a predetermined dose of one or several therapeutic useful agents to a cell, a group of cells, an organ, a tissue or an animal in which cell division is uncontrolled, such as cancer.

The expression "pharmaceutical effective amount", as used herein, is understood as an amount capable of providing a therapeutic effect, and which can be determined by the person skilled in the art by commonly used means. The amount of the polypeptide of the invention or of the functionally equivalent variant thereof, the conjugate, the polynucleotide, or the vector of the invention or of the antitumoral compound that may be combined in the pharmaceutical compositions according to the invention will vary depending upon the subject and the particular mode of administration. Those skilled in the art will appreciate that dosages may also be determined with guidance from Goodman and Goldman's The Pharmacological Basis of Therapeutics, Ninth Edition (1996), Appendix II, pp. 1707-1711 and from Goodman and Goldman's The Pharmacological Basis of Therapeutics, Tenth Edition (2001), Appendix II, pp. 475-493.

The appropriate dosage of the active principle or principles within the pharmaceutical composition will depend on the type of cancer to be treated, the severity and course of the disease, whether the composition is administered for preventive or therapeutic purposes, previous therapy, the patient's clinical history and response to the peptide or polypeptide, and the discretion of the attending physician. The amount of the polypeptide of the invention or of the functionally equivalent variant thereof, the conjugate, the polynucleotide, or a vector according to the invention, preferably carried or encapsulated by a a delivery system or encapsulation system according to the invention, is suitably administered to the patient at one time or over a series of treatments.

The pharmaceutical compositions of the invention also contain one or several additional pharmaceutically acceptable excipients. "Pharmaceutically acceptable excipient" is understood a therapeutically inactive substance said to be used for incorporating the active ingredient and which is acceptable for the patient from a pharmacological/toxicological point of view and for the pharmaceutical chemist who manufactures it from a physical/chemical point of view with respect to the composition, formulation, stability, acceptation of the patient and bioavailability.

As already stated previously, the pharmaceutical compositions of the invention comprising an agent that is a nucleic acid molecule, the nucleic acid molecule may be present within any of a variety of delivery systems known to those of ordinary skill in the art. Preferably, the nucleic acid molecule is present in a nano or micro system as defined herein. Techniques for incorporating DNA into such a delivery systema are well known to those of ordinary skill in the art.

At any rate, in general, nucleic acid molecules of the present invention may be delivered into a cell according to any one of several methods described in the art (see, e.g., Akhtar et al., Trends Cell Bio. 2:139 (1992); Delivery Strategies for Antisense Oligonucleotide Therapeutics, ed. Akhtar, 1995, Maurer et al., Mol. Membr. Biol. 16:129-40 (1999); Hofland and Huang, Handb. Exp. Pharmacol. 137:165-92 (1999); Lee et al., ACS Symp. Ser. 752:184-92 (2000); U.S. Pat. No. 6,395,713 ; International Patent Application Publication No. WO 94/02595 ); Selbo et al., Int. J. Cancer 87:853-59 (2000); Selbo et al., Tumour Biol. 23:103-12 (2002); U.S. Patent Application Publication Nos. 2001/0007666 , and 2003/077829 ). Such delivery methods known to persons having skill in the art, include, but are not restricted to, encapsulation in liposomes, nano or micro systems (as indicated in the present examples), by iontophoresis, or by incorporation into other vehicles, such as biodegradable polymers; hydrogels; cyclodextrins (see, e.g., Gonzalez et al., Bioconjug. Chem. 10: 1068-74 (1999); Wang et al., International Application Publication Nos. WO 03/47518 and WO 03/46185); poly(lactic-co-glycolic)acid (PLGA) and PLCA microspheres (also useful for delivery of peptides and polypeptides and other substances) (see, e.g., U.S. Pat. No. 6,447,796 ; U.S. Patent Application Publication No. 2002/130430 ); biodegradable nanocapsules; and bioadhesive microspheres, or by proteinaceous vectors (International Application Publication No. WO 00/53722 ).

In a preferred embodiment, the pharmaceutical composition or pharmaceutical combination according to the invention further comprises together or separately an antitumoral agent.

As used herein, "antitumoral agent" is understood as said biological or chemical compound which treats tumors or prevents the formation thereof. In a preferred embodiment said antitumoral agent is selected from the group consisting of a cytotoxic agent, an antiangiogenic agent, an antimetastatic agent and an antiproliferative agent.

The pharmaceutical compositions or combinations of the invention can be administered by any type of suitable route, such as by oral route, topical route, by inhalation or parenteral route so that the pharmaceutically acceptable excipients necessary for the formulation of the desired dosage form will be included.

In another aspect, the invention relates to a polypeptide or functional equivalent variant of said polypeptide according to the invention, a conjugate according to the invention, a polynucleotide according to the invention, a vector according to the invention, preferably encapsulated or carry in a delivery system of the invention, or a pharmaceutical composition according to the invention for use in medicine.

In another aspect, the invention relates to a polypeptide or functional equivalent variant of said polypeptide according to the invention, a conjugate according to the invention, a polynucleotide according to the invention, a vector according to the invention, preferably encapsulated or carry in a delivery system of the invention, or a pharmaceutical composition according to the invention for use in the prevention and/or treatment of cancer.

Alternatively, the invention relates to a method for preventing and/or treating cancer (preferably as any treatment which is designed to cure, alleviate, remove or lessen the symptoms of, or prevent or reduce the possibility of contracting cancer) comprising administering to a subject in need thereof a therapeutically effective amount of a polypeptide or functional equivalent variant of said polypeptide according to the invention, a conjugate according to the invention, a polynucleotide according to the invention, a vector according to the invention, preferably encapsulated or carry in a delivery system of the invention, or a pharmaceutical composition according to the invention.

Alternatively, the invention relates to the use of a polypeptide or functional equivalent variant of said polypeptide according to the invention, a conjugate according to the invention, a polynucleotide according to the invention, a vector according to the invention, preferably encapsulated or carry in a delivery system of the invention, or a pharmaceutical composition according to the invention for the preparation of a medicament for the prevention and/or treatment of cancer (preferably as any treatment which is designed to cure, alleviate, remove or lessen the symptoms of, or prevent or reduce the possibility of contracting cancer).

"Prevention" is understood as the administration of a compound in an initial or early stage of the disease, or to also prevent its onset.

The term "treatment" is used to designate the administration of a compound to control the progression of the disease before or after the clinical signs have appeared. Control of the progression of the disease is understood as the beneficial or desired clinical results which include but are not limited to reduction of the symptoms, reduction of the duration of the disease, stabilization of pathological conditions (specifically avoiding additional impairment), delaying the progression of the disease, improving the pathological condition and remission (both partial and complete). The control of the progression of the disease also involves a prolongation of survival in comparison to the expected survival if the treatment was not applied.

A "subject," as used herein, includes any animal that has a cancer or exhibits a symptom or cancer, or is at risk for having a cancer or exhibiting a symptom of cancer. Suitable subjects (patients) include laboratory animals (such as mouse, rat, rabbit, or guinea pig), farm animals, and domestic animals or pets (such as a cat or dog). Non-human primates and, preferably, human patients, are included.

The term "cancer" is referred to a disease characterized by uncontrolled cell division (or by an increase of survival or apoptosis resistance), by the ability of said cells to invade other neighbouring tissues (invasion) or by the spread to other areas of the body where the cells are not normally located (metastasis) through the lymphatic and blood vessels. Depending on whether or not tumours can spread by invasion and metastasis, they are classified as being either benign or malignant: benign tumours are tumours that cannot spread by invasion or metastasis, i.e., they only grow locally; whereas malignant tumours are tumours that are capable of spreading by invasion and metastasis. The methods according to the present invention are useful for the treatment of local and malignant tumours. As used herein, the term cancer includes, but is not limited to, the following types of cancer: breast cancer; biliary tract cancer; bladder cancer; brain cancer including glioblastomas and medulloblastomas; cervical cancer; choriocarcinoma; colon cancer; endometrial cancer; esophageal cancer; gastric cancer; hematological neoplasms including acute lymphocytic and myelogenous leukemia; T-cell acute lymphoblastic leukemia/lymphoma; hairy cell leukemia; chronic myelogenous leukemia, multiple myeloma; AIDS-associated leukemias and adult T-cell leukemia/lymphoma; intraepithelial neoplasms including Bowen's disease and Paget's disease; liver cancer; lung cancer; lymphomas including Hodgkin's disease and lymphocytic lymphomas; neuroblastomas; oral cancer including squamous cell carcinoma; ovarian cancer including those arising from epithelial cells, stromal cells, germ cells and mesenchymal cells; pancreatic cancer; prostate cancer; rectal cancer; sarcomas including leiomyosarcoma, rhabdomyosarcoma, liposarcoma, fibrosarcoma, and osteosarcoma; skin cancer including melanoma, Merkel cell carcinoma, Kaposi's sarcoma, basal cell carcinoma, and squamous cell cancer; testicular cancer including germinal tumors such as seminoma, non-seminoma (teratomas, choriocarcinomas), stromal tumors, and germ cell tumors; thyroid cancer including thyroid adenocarcinoma and medullar carcinoma; and renal cancer including adenocarcinoma and Wilms tumor. Other cancers will-be known to one of ordinary skill in the art. In a preferred embodiment, the cancer treated is breast cancer.

In a preferred embodiment the cancer is a solid tumor. In preferred embodiment, the polypeptide or functional equivalent variant of said polypeptide according to the invention, the conjugate according to the invention, the polynucleotide according to the invention, the vector according to the invention, or the pharmaceutical composition according to the invention, is for use in a method of treatment of a solid tumor, preferably a tumor selected from the following group: breast cancer, pancreatic cancer, lung cancer, prostate cancer, stomach cancer, head and neck cancer, sarcoma, glioblastoma, neuroblastoma, cancer of the colon and rectum, cancer of the head and neck, kidney and bladder cancer, and hepatocarcinoma. Preferably, the solid tumour is a breast tumour.

In preferred embodiment, the polypeptide or functional equivalent variant of said polypeptide according to the invention, the conjugate according to the invention, the polynucleotide according to the invention, the vector according to the invention, preferably encapsulated or carry in a delivery system of the invention, or the pharmaceutical composition according to the invention, is for use in a method of treatment of cancer metastasis, preferably a solid tumour or cancer metastasis originated from a solid tumor, preferably a tumor selected from the following group: breast cancer, gynecological, pancreas, lung cancer, prostate cancer, stomach cancer, head and neck cancer, sarcoma, glioblastoma, neuroblastoma, cancer of the colon and rectum, cancer of the head and neck, kidney and bladder cancer, and hepatocarcinoma. Preferably, the solid tumour is a breast tumour.

The invention is detailed below by means of the following examples which are merely illustrative and by no means limiting for the scope of the invention.

### BRIEF DESCRIPTION OF THE FIGURES

**Figure 1****.** Graphical representation of *in vivo* antitumour evaluation.
**Figure 2****.** Followed strategy based on the intracellular delivery of GSDM-B-NT to treat TNBC cells making use of HA-coated putrescine nanosystems.
**Figure 3****.** Scanning transmission electron microscope images of antitumour-PSN before and after coating with HA. Scale bars = 400 nm.
**Figure 4****.** Stability of HA-coated PSN upon incubation with complete cellular medium in terms of (a) mean hydrodynamic size and (b) Pdl
**Figure 5****.** Stability of HA-coated PSN upon incubation with human plasma in terms of (a) mean hydrodynamic size, (b) polydispersity index (Pdl), and (c) zeta potential (ZP)
**Figure 6****.** *In vitro* assays carried out in MDA-MB-231 cell line with the aim of evaluating (a) the cellular internalization of lytic-PSN-HA (blue: DAPI, green: TopFluor-labelled nanosystems) and the anticancer effect of lytic-PSN compared to empty-PSN by (a) Alamar Blue and (b) Annexin V-FITC (A+) plus propidium iodide (PI+) staining measured by flow cytometry. A+ cells are represented in grey, whereas A+/PI+ cells are represented in purple. Statistical significance was determined by two-tailed unpaired t-test (^{∗}P<0.05; ^{∗∗}P<0.01). Data are shown as mean ± SEM.
**Figure 7****.** *In vivo* evaluation carried out in mice bearing MDA-MB-231 breast cancer tumours. (a) *Ex vivo* biodistribution of TopFluor-labelled control-PSN, after intraperitoneal treatment, determined by TopFluor fluorescence (increasing signal from black to yellow) (n=1/group); (b) Tumour inhibition growth evaluated in terms of growth volume. Grey line shows the beginning of the treatment (n≥3/group). Statistical significance was determined by two-tailed unpaired t-test (·P<0.1; ^{∗}P<0.05).

### EXAMPLES

### 1. MATERIALS AND METHODS

### 1.1 Materials

DI-α-tocopherol (Vitamin E), water nuclease free, and Mowiol^{®} were supplied by Merck Millipore (Spain). Egg sphingomyelin (SM) was bought from Lipoid GmbH (Ludwigshafen, Germany). C11 TopFluor Sphingomyelin (N-[11-(dipyrrometheneboron difluoride) undecanoyl]-D-erythro-sphingosyl-phosphorylcholine was obtained from Avanti Polar Lipids (Alabaster, AL, USA). Analytical grade solvents were purchased from VWR (Barcelona, Spain) or Galmedic (A Coruña, Spain). The lipidic putrescine, (9Z)-N-(4-Aminobutyl)-9-octadecenamide, was synthesized by Galchimia SA (Galicia, Spain). Sodium hyaluronate was supplied by Lifecore Biomedical^{™}. MDA-MB-231 and SK-BR-3 breast cancer cell lines were purchased from the American Type Culture Collection (ATCC).

### 1.2 Plasmid generation

The 1-275 NT domain of GSDM-B, previously reported by Ding J *et al.* [7] was cloned with the pLVX vector from Mutagenex (Ohio State University (OSU), Columbus, Ohio). Basically, the cDNA obtained was cloned in the lentiviral vector pLVX following the Ligase T4 DNA protocol (Invitrogen). Plasmid transformation was performed in DH5*α* bacteria following the conventional thermal shock transformation protocol. cDNA was isolated by High Pure Plasmid Isolation Kit (Roche) and all plasmids were verified by DNA SANGER sequencing.

### 1.3 Preparation of the nanosystems

As previously described, putrescine nanosystems were formulated by ethanol injection method. Briefly, 5 mg of vitamin E (VitE), 0.5 mg of sphingomyelin (SM) and 0.25 mg of putrescine modified with oleamide (Pt) were dissolved in 100 µl of ethanol and injected in 1 ml of Molecular Grade Water. Nanosystems were instantaneous formed. Then, 5 µg of pDNA were dissolved in 100 µL of H₂O nuclease-free and added over 100 µL of preformed nanocarriers, for 20 min under stirring to achieve the association. Next, 20µL of sodium hyaluronate at 1 mg/mL were added to the nanosystems, and those were stirred for 5 min to achieve the coating.

### 1.4 Physicochemical characterization

The nanocarriers were characterized using a Zetasizer^{®} (NanoZS Malvern Instruments, England) to determine the particle size and polydispersity index (Pdl) by dynamic light scattering (DLS). All samples were analysed after dilution by 10-fold with MilliQ water in disposable microcuvettes (ZEN0040), at room temperature, and with a detection angle of 173º. Moreover, the Z potential (ZP), indicative of the surface charge of the nanocarriers was determined using the same instrument, by Laser Doppler anemometry (LDA) after dilution by 40-fold with MilliQ water, in Folded capillary cells cuvettes (DTS 1070, Malvern Instruments). Each sample is given by 3 measurements and the average of independent samples is provided (n = 3).

Morphological examination was performed by Scanning transmission electron microscopy (STEM) using a Field Emission Scanning Electron Microscopy (FESEM) Ultra Plus (Zeiss, Germany) operating at 20 kV. Nanosystems were diluted 5-fold, and 20 µl of them were mixed with 20 µl of phosphotungstic acid 2% (w/v) to stain. The mixture was placed in a copper grid for 6 hours, washed with 500 µl of water and dried overnight under vacuum.

### 1.5 Stability evaluation

Human peripheral blood was obtained by direct venepuncture in CellSave tubes (Menarini, Silicon Biosystems, Bologna, Italy) and processed within 96 h after blood collection. Plasma and cellular components were separated by centrifugation at 1,600 g for 10 minutes at room temperature. Next, plasma was centrifugated again at 5,500 g for 10 minutes at room temperature in order to remove any remaining cellular debris. Human plasma was then diluted 1:10 with MilliQ water and filtered with a 0.22 µm low protein binding (PVDF) filter. Finally, nanosystems were incubated at 1:10 dilution and directly measured by DLS. Additionally, stability upon incubation with complete cellular medium was evaluated by measuring nanosystems diluted 7-fold with DMEM supplemented with 1% FBS.

### 1.6 Cell Culture

MDA-MB-231 triple negative breast cancer cell line was obtained from the American Type Cell Culture (ATCC). Cells were cultured following the supplier conditions. Briefly, MDA-MB-231 cells were cultured in Dulbecco's modified Eagle's medium (DMEM, Gibco) supplemented with 10% Fetal bovine serum (Gibco), 2% Amphotericin-B (Gibco), 10 mM glutamine (Gibco), and 1% penicillin/streptomycin (Invitrogen). Cells were maintained at 37ºC, with 95% relative humidity and 5% CO₂. In addition, mCherry-luciferase transfected cells, previously obtained by lentiviral infection [20], were used for *in vivo* assays.

### 1.7 Cellular viability assays

Cellular viability after treatment with the nanosystems was evaluated using the AlamarBlue^{™} reagent (Bio-Rad, Madrid, Spain) according to the manufacturer's instructions. Briefly, 10.000 cells per well were seeded on 96-well plate in triplicates. After 24 h, cells were treated with different concentrations of nanocarriers (0.25 and 0.5 mg/mL in medium without supplement) for 4h. Then, medium was removed, and fresh completed medium was added for 48h. Spectrophotometric analysis was performed at wavelengths of 570 and 600 nm with a Synergy HT reader (BioTek Instruments). At least three independent experiments were carried out.

### 1.8 Cell apoptosis analysis

Cell death rate was quantified by flow cytometry using the Annexin V-FITC stained apoptosis detection kit (Immunostep, Salamanca Spain) and propidium iodide (PI, Sigma-Aldrich) according to the manufacturer's instructions. First, 190.000 cells were seeded per well on 6-well plates. Cancer cells were incubated for 4h with the nanosystems at 0.5 mg/ml with non-supplemented medium. After that, fresh completed medium was added, and cells were incubated for 48 additionally hours. The apoptosis analysis was conducted using a FC500 flow cytometer (Beckman Coulter, California, USA), by evaluating 10.000 events per condition. Three independent experiments were performed.

### 1.9 In vitro time lapse

MDA-MB-231 were seeded on lbidi µ-Slide 8 well plates (40.000 cells/well). After 24h, empty-PSN and lytic-PSN were added to the cells at 0.5 mg/mL with non-supplemented medium. Living cells were photographed with Cell Observer^{®} (Zeiss, Germany) microscope and pictures were taken as follows: Each 5 min for 30 min, each 15 min for 90 min, and each 30 min for 3 h.

### 1.10 In vivo evaluation

All the *in vivo* experiments were conducted in the animal facilities of "Alberto Sols" Biomedical Research Institute (IIB), approved by the internal ethical research and animal welfare committee (IIB, UAM), and by the Local Authorities (Comunidad de Madrid, PROEX235.6/20). Procedures followed by the European Union (Directive 2010/63/UE) and Spanish Government guidelines (Real Decreto 53/20133).

Orthotropic breast tumours were generated by injecting 1 × 10⁶ MDA-MB-231 cells in 100 µL PBS into the left fifth mammary fat pad (mfp) of 7-week-old female nu/nu mice (Charles River) as previously described [19,27].

### 1.10.1. Biodistribution assays

After tumour generation, a proof of concept was performed to evaluate the biodistribution of the nanosystems, treating for that purpose, one mouse per condition. With this aim, fluorescent-labelled empty-PSN-HA were intraperitoneally administered at 52.5 mg/kg of lipids and 0.5 mg/kg of pDNA per mice. As a control, one mouse did not receive any treatment. After 8h and 24h of treatment, mice were anesthetized and sacrificed. Then, organs were carefully removed and analysed by the *In Vivo* Imaging Systems IVIS^{®}.

### 1.10.2. Therapeutic effect

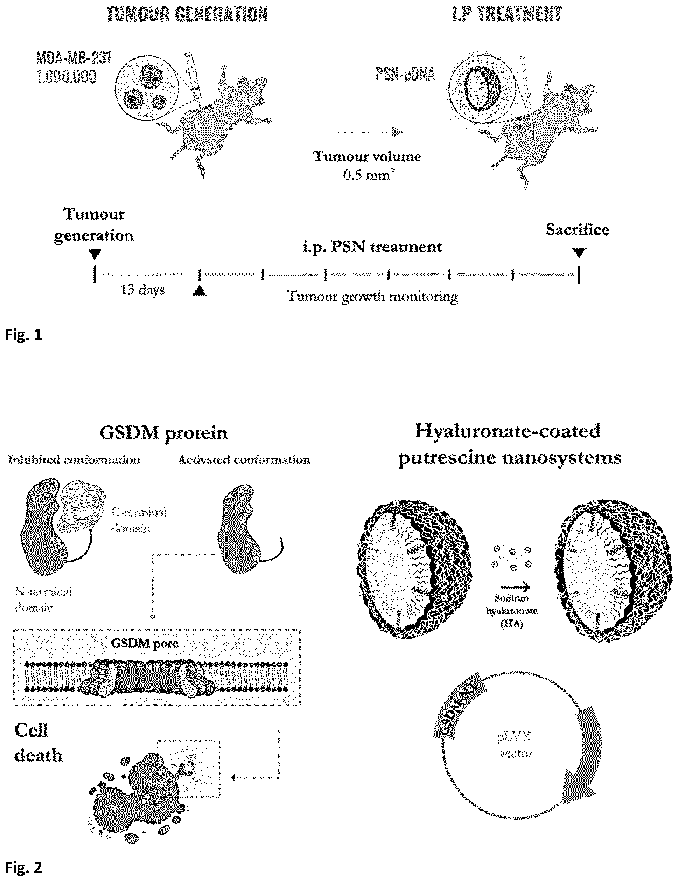

Once tumours have acquired an average volume of 0.5 mm, three mice per condition were intraperitoneally treated with control-PSN or antitumour-PSN at 0.5 mg/kg of pDNA. Next, tumour growth was monitored by calibre rule and luciferase bioluminescence emission, prior retroorbital injection. At the end of the experiments, organs were removed, and analysed by IVIS^{®}. Experimental design is scheduled in Figure 1.

### 1.11. Statistical analysis

A Shapiro-Wilk test was carried out to evaluate the normality of the samples. After confirming that, two-tailed unpaired t-tests were performed. A P value < 0.05 was considered as statistically significant.

### 2. RESULTS

Intracellular delivery of the N-terminal domain of GSDM-B has shown a high ability to decrease cellular viability [7]. However, effective carriers are needed to achieve this goal, which lack has limited their use to develop novel therapeutic agents. Putrescine nanosystems (PSN) have demonstrated to be effective non-viral vectors with promising results in the intracellular delivery of nucleic acids, showing a significant decrease of triple negative breast tumour growth in zebrafish embryo and mice models. Thus, PSN have been employed to associate a plasmid encoding for the N-terminal domain of GSDM-B as well as, the empty vector (pLVX), namely lytic-PSN and empty-PSN, respectively. Nanosystems were subsequently coated with hyaluronic acid and their ability to treat triple negative breast cancer cells was investigated (Figure 2).

With this in frame, PSN were prepared by the ethanol injection method, and fully characterized. The average size distribution had a mean size around 90 nm and a highly positive zeta potential (Table 1) due to the presence of putrescine in the composition. Association of the plasmid encoding for the NT domain of GSDM-B (Lytic-PSN), or the empty vector pLVX (empty-PSN) employed as a control, resulted in an increase in size formulations with average hydrodynamic sizes of 130 and 163 nm, with low polydispersity indexes (PdI) value, and positive zeta potentials (Table 1). These physiochemical changes, that is, an increase in size and a decrease in the zeta potential suggested an efficient association of the plasmids, and demonstrating the versatility of PSN to associate a variety of plasmids without being seriously affected by their size. In this sense, small differences were observed with respect to the type of plasmid. Nanosystems loaded with the plasmid of interest were larger in size, which could be due to its higher size as it contains the insert of the gene of interest (8981 bp), with respect to the control sequence (8012 bp).

The next step was to coat the plasmid-loaded PSNs with HA, a major component of the extracellular matrix widely used for gene therapy applications [22,28,29]. With this aim, a solution of HA of 151-300 KDa was added into the suspension of the nanosystems, giving rise to empty-PSN-HA and lytic-PSN-HA. After coating, it was observed an increase in size of approximately 50 nm, while the PdI was maintained. Importantly, HA-coating led to an inversion in the zeta potential (Table 1), an indicative of a successful surface functionalization.

**Table 1. Association of plasmids and effect of coating with sodium hyaluronate (HA) on the physicochemical characteristics. Mean ± SD (n≥3).**

| **Formulation** | **Size (nm)** | **PdI^{a}** | **ZP^{b} (mV)** |
|---|---|---|---|
| PSN | 87 ± 9 | 0.23 | + 62 ± 8 |
| Empty-PSN | 130 ± 4 | 0.15 | + 37 ± 4 |
| Lytic-PSN | 163 ± 4 | 0.14 | + 33 ± 2 |
| Empty-PSN-HA | 182 ± 3 | 0.15 | - 25 ± 1 |
| Lytic-PSN-HA | 202 ± 7 | 0.14 | - 26 ± 1 |

| | | | |
|---|---|---|---|
| ^{a}PdI: Polydispersity Index; ^{b}ZP: Zeta Potential | | | |

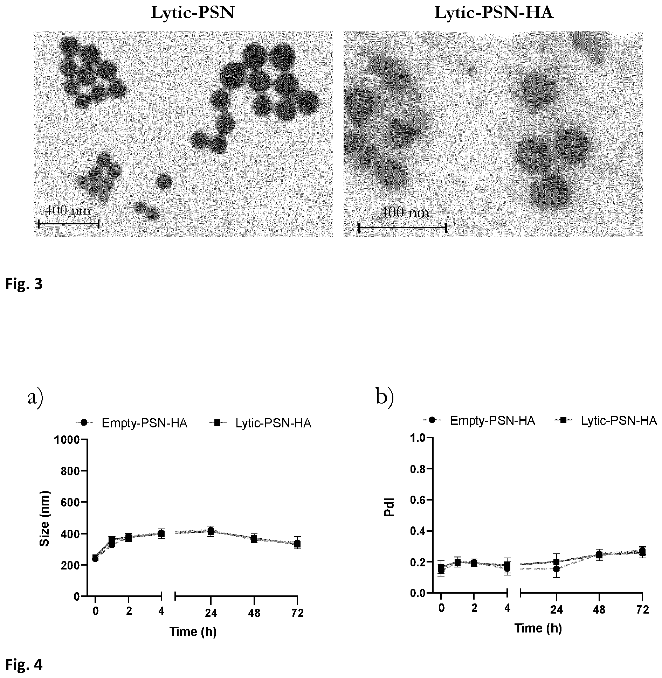

Evidence of a successful coating has also been obtained upon morphological evaluation. As observed in Figure 3, whereas non-coated nanosystems show a well-defined spherical shape, coated nanosystems exhibit a polymeric surface layer. Non-coated nanosystems have shown an almost identical patron to other putrescine complexes, regarding morphological appearance [30].

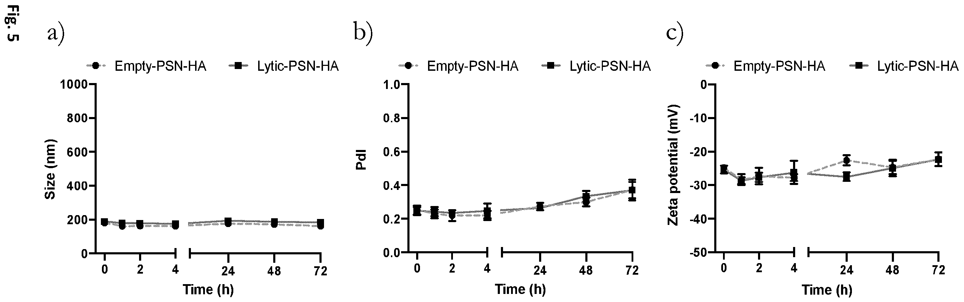

Next experiments were performed to assess the stability of HA-coated pDNA-loaded nanosystems upon incubation with different complex media. Upon incubation with complete cellular medium, both empty-PSN-HA and lytic-PSN-HA suffered an increase in size of around 200 nm after 1 h of incubation (Figure 4), most probably, because of the surrounding by proteins from serum [31]. Importantly, PdI was maintained after 72 h. These findings are in agreement with previous stability studies which have shown a dramatic increase in size after 1 h of incubation [31]. In fact, previous PSN-pDNA have shown same results, suggesting that, regarding their stability, the coating has not provided significant advantages, contrary to its reported ability to avoid protein adsorption [32]. Second, stability upon incubation with human plasma was also tested. In this case, size and zeta potential were maintained, with only a slight increase of Pdl after 72 h (Figure 5). This better behaviour, after human plasma incubation compared to the supplemented cellular medium, has been already observed for sphingomyelin nanosystems [33]. Remarkably, this high stability is of great importance for future *in vivo* applications.

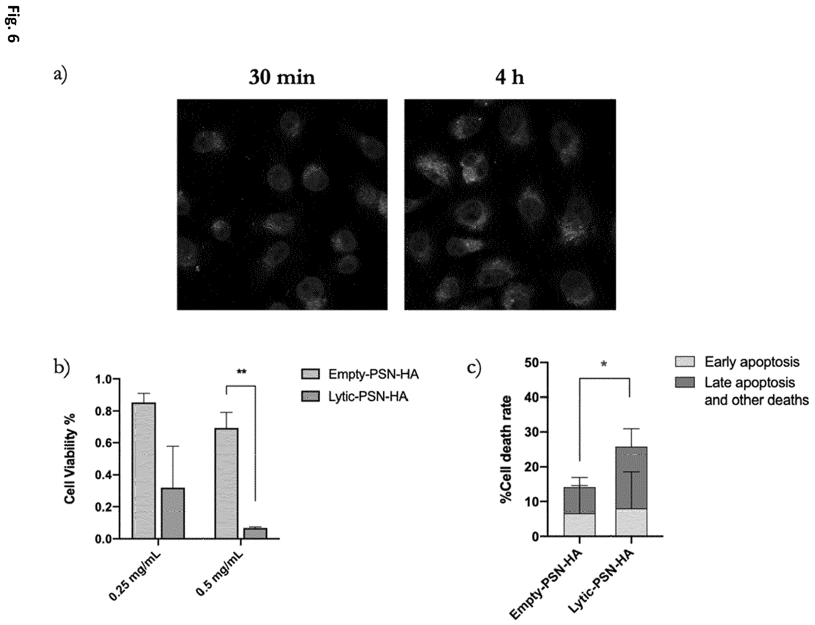

After having characterized the HA-coated pDNA-loaded PSN, their cellular internalization was evaluated in MDA-MB-231, a triple negative breast cancer cell line. For this purpose, the nanosystems were fluorescently labelled with sphingomyelin-TopFluor^{®}. Upon incubation with the fluorescent nanosystems (lytic-PSN-HA), cells were analysed at two different time points by confocal microscopy. In view of the results, nanosystems are quickly internalized by cancer cells, which have shown a weak signal after only 30 minutes of treatment. Importantly, after 4 h of treatment, all cells have internalized the nanosystems (Figure 6a).

Therapeutic activity was further evaluated by measuring the decrease on the cellular viability. In view of the results, nanosystems were incubated for 4 h, after which time, fresh medium was added, and cells were kept for 48 h. Our findings revealed, at first place, that the formulation with the empty vector, (empty-PSN-HA), did not induce toxic effects in cells. Oppositely, the formulation carrying the plasmid encoding for the NT domain of GSDM-B (lytic-PSN-HA) lead to a decrease on cell viability, especially when 0.5 mg/mL of the lytic treatment was applied (Figure 6b). Moreover, it was also observed a concentration-dependent profile. In comparison to the study of reference [7], although it cannot be directly compared due to the technical differences (cell line, strategy, time, concentration), a higher decreased in the cellular viability was observed here. Whereas a cellular viability of 20 % was observed after treating human 293T cells [7], the delivery of the GSDM-B-NT into the MDA-MB-231, using the developed nanosystems has led to an average cellular viability of 8.4 %. Importantly, this results significantly improves the previously developed gene therapy, based on the delivery of a plasmid encoding for the protein Fas Ligand, which have not shown any reduction on the cellular viability at 0.5 mg/mL. In fact, the significant drop observed in this experiment has not been observed even with a dose of 1 mg/mL at higher times of treatment, which highlights the potential of the developed nanotherapy. Likewise, the compromised cellular viability after treatment with the lytic-PSN-HA formulation was confirmed by measuring cell staining with propidium iodide and Annexin V, by flow cytometry (Figure 6c).

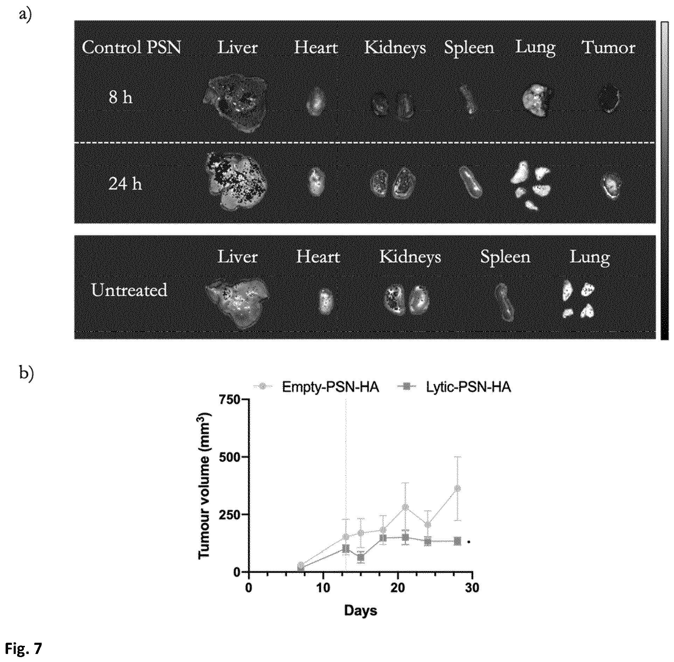

Finally, an *in vivo proof-of-concept* was performed in TNBC mice model bearing orthotopic tumours. First, the biodistribution assay was carried out after 8 and 24 h after treatment of the control PSN intraperitoneally (i.p) inoculated. As observed in Figure 7a, in view of TopFluor-labelled nanosystems signal, nanosystems are partially accumulated in the liver after 8 h, however, a great accumulation was observed in the tumour, which was considerably increased after 24 h of treatment. Several factors may favour this tumour preference. First, slightly negative nanosystems have been observed to better accumulate into the tumours, with higher circulation half-life compared to positively-charged nanosystems [35]. Second, both putrescine and hyaluronic acid, components of the final formulation, have demonstrated their enhanced interaction with cancer cells [23,24,32,36]. However, further studies must be performed to better understand this behaviour, as well as evaluate other possible administration routes. An increase of signal due to the TopFluor-labelled nanosystems was also observed in the kidneys, most probably consequence of renal clearance, as reported by previous studies which evaluate the biodistribution of sphingomyelin nanosystems after i.v. injection [34,37]. Importantly, fluorescence signal in the heart, which would be a symptom of later cardiotoxicity, was not found. In our study, the fluorescence signal has mainly shown liver accumulation, without significant signal in other organs [38]. Likewise, the injection of other nanosystems after 8 h have also shown delivery mainly into the liver and kidneys [39].

Finally, a therapeutic experiment was carried out. The effect was evaluated after 6 repeated doses of lytic-PSN-HA, with a dose of 10 µg pDNA / mice / day, as summarized in Figure 1. PSNs loaded with the empty vector (empty-PSN-HA) were used for control.

Importantly, mice receiving the treatment formulation (lytic-PSN-HA) showed a reduction in the tumour growth, with respect to mice receiving the control formulation. At the end of the experiment, an evident tendency was observed, highlighted by the low P-value (0.0595) (Figure 7b). Similar results have been previously obtained after treating 0.5 mm³ (same initial point) TNBC tumours in BALB/c mice with HA-coated nanosystems loading a plasmid which encodes for the tumour necrosis factor-related apoptosis-inducing ligand (TRAIL) [40]. Remarkably, the dose used in this study doubles the dose evaluated in our case.

This pilot study is of great importance not only because of the result, but also because of the possibility to carryout it, representing the first reported *in vivo* evaluation of intracellularly deliver the GSDMB-NT. It has been possible because of the developed nanosystems, which provide a non-viral vector able to associate pDNAs and successfully achieve its delivery into the breast tumour. Overall, it proofs the potential of nanomedicine, and particularly, of putrescine nanosystems, to provide innovative opportunities for triple negative breast cancer treatment.

Next experiments will be carried out to confirm the activation of immunogenic cell death, and particularly pyroptotic pathways, as well as to study the potential of the developed nanosystems to treat metastasis or other cancer types. In addition, the ability of PSN to associate other type of nucleic acids, such as miRNA will be studied to confirm its versatility as non-viral vectors for gene therapy applications.

### REFERENCES

[1] D. Sarrió, J. Martínez-Val, Á. Molina-Crespo, L. Sánchez, G. Moreno-Bueno, The multifaceted roles of gasdermins in cancer biology and oncologic therapies, Biochim. Biophys. Acta - Rev. Cancer. 1876 (2021). https://doi.org/10.1016/j.bbcan.2021.188635.
[2] C. Fu, Gasdermin: a novel therapeutic target for tumour treatment by activating anti-tumour immunity, Signal Transduct. Target. Ther. 5 (2020) 3-4. https://doi.org/10.1038/s41392-020-0180-4.
[3] X.-X. Liao, Y.-Z. Dai, Y.-Z. Zhao, K. Nie, Gasdermin E: A Prospective Target for Therapy of Diseases, Front. Pharmacol. 13 (2022) 1-16. https://doi.org/10.3389/fphar.2022.855828.
[4] Y. Wang, J. Peng, X. Xie, Z. Zhang, M. Li, M. Yang, Gasdermin E-mediated programmed cell death: An unpaved path to tumor suppression., J. Cancer. 12 (2021) 5241-5248. https://doi.org/10.7150/jca.48989.
[5] J. Ibrahim, E. De Schutter, K. Op de Beeck, GSDME: A Potential Ally in Cancer Detection and Treatment, Trends in Cancer. 7 (2021) 392-394. https://doi.org/10.1016/j.trecan.2020.12.002.
[6] Y. Lu, W. He, X. Huang, Y. He, X. Gou, X. Liu, Z. Hu, W. Xu, K. Rahman, S. Li, S. Hu, J. Luo, G. Cao, Strategies to package recombinant Adeno-Associated Virus expressing the N-terminal gasdermin domain for tumor treatment, Nat. Commun. 12 (2021). https://doi.org/10.1038/s41467-021-27407-0.
[7] J. Ding, K. Wang, W. Liu, Y. She, Q. Sun, J. Shi, H. Sun, D.C. Wang, F. Shao, Pore-forming activity and structural autoinhibition of the gasdermin family, Nature. 535 (2016) 111-116. http s: / /doi.org/ 10.1038/nature 18590.
[8] J. Zou, Y. Zheng, Y. Huang, D. Tang, R. Kang, R. Chen, The Versatile Gasdermin Family: Their Function and Roles in Diseases, Front. Immunol 12 (2021) 1-17. https://doi.org/10.3389/fimmu.2021.751533.
[9] X. Liu, Z. Zhang, J. Ruan, Y. Pan, V.G. Magupalli, H. Wu, J. Lieberman, Inflammasome - activated gasdermin D causes pyroptosis by forming membrane pores, Nature. 535 (2016) 153-158. https://doi.org/10.1038/nature18629.Inflammasome.
[10] C.B. Ryder, H.C. Kondolf, M.E. O'Keefe, B. Zhou, D.W. Abbott, Chemical Modulation of Gasdermin-Mediated Pyroptosis and Therapeutic Potential, J. Mol. Biol. 434 (2022) 167183. https://doi.org/10.1016/j.jmb.2021.167183.
[11] G. Sollberger, A. Choidas, G.L. Burn, P. Habenberger, R. Di Lucrezia, S. Kordes, S. Menninger, J. Eickhoff, P. Nussbaumer, B. Klebl, R. Krüger, A. Herzig, A. Zychlinsky, Gasdermin D plays a vital role in the generation of neutrophil extracellular traps, Sci. Immunol. 3 (2018). https://doi.org/10.1126/sciimmunol.aar6689.
[12] B.T. Cookson, M.A. Brennan, Pro-inflammatory programmed cell death., Trends Microbiol. 9 (2001) 113-114. https://doi.org/10.1016/s0966-842x(00)01936-3.
[13] P. Yu, X. Zhang, N. Liu, L. Tang, C. Peng, X. Chen, Pyroptosis: mechanisms and diseases, Signal Transduct. Target. Ther. 6 (2021). https://doi.org/10.1038/s41392-021-00507-5.
[14] Y.Y. Wang, X.L. Liu, R. Zhao, Induction of pyroptosis and its implications in cancer management, Front. Oncol. 9 (2019) 1-10. https://doi.org/10.3389/fonc.2019.00971.
[15] Y. Fang, S. Tian, Y. Pan, W. Li, Q. Wang, Y. Tang, T. Yu, X. Wu, Y. Shi, P. Ma, Y. Shu, Pyroptosis: A new frontier in cancer, Biomed. Pharmacother. 121 (2020). https://doi.org/10.1016/j.biopha.2019.109595.
[16] D. Wu, C. Wei, Y. Li, X. Yang, S. Zhou, Pyroptosis, a New Breakthrough in Cancer Treatment, Front. Oncol. 11 (2021) 1-15. https://doi.org/10.3389/fonc.2021.698811.
[17] S. Elmore, Apoptosis: a review of programmed cell death., Toxicol. Pathol. 35 (2007) 495-516. https://doi.org/10.1080/01926230701320337.
[18] N. Saeki, Y. Kuwahara, H. Sasaki, H. Satoh, T. Shiroishi, Gasdermin (Gsdm) localizing to mouse chromosome 11 is predominantly expressed in upper gastroiatestinal tract but significantly suppressed in human gastric cancer cells, Mamm. Genome. 11 (2000) 718-724. https://doi.org/10.1007/s003350010138.
[19] M. Hergueta-Redondo, D. Sarrió, Á. Molina-Crespo, D. Megias, A. Mota, A. Rojo-Sebastian, P. Garcia-Sanz, S. Morales, S. Abril, A. Cano, H. Peinado, G. Moreno-Bueno, Gasdermin-B promotes invasion and metastasis in breast cancer cells, PLoS One. 9 (2014). https://doi.org/10.1371/journal.pone.0090099.
[20] A. Molina-Crespo, A. Cadete, D. Sarrio, M. Gamez-Chiachio, L. Martinez, K. Chao, A. Olivera, A. Gonella, E. Diaz, J. Palacios, P.K. Dhal, M. Besev, M. Rodriguez-Serrano, M.L.G. Bermejo, J.C. Triviño, A. Cano, M. Garcia-Fuentes, O. Herzberg, D. Torres, MJ. Alonso, G. Moreno-Bueno, Intracellular delivery of an antibody targeting gasdermin-b reduces her2 breast cancer aggressiveness, Clin. Cancer Res. 25 (2019) 4846-4858. https://doi.org/10.1158/1078-0432.CCR-18-2381.
[21] M. Hergueta-Redondo, D. Sarrio, Á. Molina-Crespo, R. Vicario, C. Bernadó-Morales, L. Martinez, A. Rojo-Sebastián, J. Serra-Musach, A. Mota, Á. Martinez-Ramirez, M.Á. Castilla, A. González-Martin, S. Pernas, A. Cano, J. Cortes, P.G. Nuciforo, V. Peg, J. Palacios, M.Á. Pujana, J. Arribas, G. Moreno-Bueno, Gasdermin B expression predicts poor clinical outcome in HER2-positive breast cancer, Oncotarget. 7 (2016) 56295-56308. https://doi.org/10.18632/oncotarget.10787.
[22] M. De La Fuente, B. Seijo, MJ. Alonso, Design of novel polysaccharidic nanostructures for gene delivery, Nanotechnology. 19 (2008). https://doi.org/10.1088/0957-4484/19/7/075105.
[23] M.J. Santander-Ortega, M. De La Fuente, M. V. Lozano, M.E. Bekheet, F. Progatzky, A. Elouzi, I.F. Uchegbu, A.G. Schätzlein, Hydration forces as a tool for the optimization of core-shell nanoparticle vectors for cancer gene therapy, Soft Matter. 8 (2012) 12080-12092. https://doi.org/10.1039/c2sm26389k.
[24] M. Hornof, M. de la Fuente, M. Hallikainen, R.H. Tammi, A. Urtti, Low molecular weight hyaluronan shielding of DNA/PEI polyplexes facilitates CD44 receptor mediated uptake in human corneal epithelial cells, J. GeneMed. 10 (2008) 70-80. https://doi.org/10.1002/jgm.1125.
[25] M. Raviña, E. Cubillo, D. Olmeda, R. Novoa-carballal, E. Femandez-megia, R. Riguera, Hyaluronic Acid / Chitosan-g-Poly ( ethylene glycol) Nanoparticles for Gene Therapy : An Application for pDNA and siRNA Delivery, Pharm. Res. 27 (2010) 2544-2555. https://doi.org/10.1007/s11095-010-0263-y.
[26] G. Mattheolabakis, L. Milane, A. Singh, M.M. Amiji, Hyaluronic acid targeting of CD44 for cancer therapy: From receptor biology to nanomedicine, J. Drug Target. 23 (2015) 605-618. https://doi.org/10.3109/1061186X.2015.1052072.
[27] D. Olmeda, G. Moreno-Bueno, J.M. Flores, A. Fabra, F. Portillo, A. Cano, SNAI1 is required for tumor growth and lymph node metastasis of human breast carcinoma MDA-MB-231 cells, Cancer Res. 67 (2007) 11721-11731. https://doi.org/10.1158/0008-5472.CAN-07-2318.
[28] J.H. Kim, M.J. Moon, D.Y. Kim, S.H. Heo, Y.Y. Jeong, Hyaluronic acid-based nanomaterials for cancer therapy, Polymers (Basel). 10 (2018) 1-15. https://doi.org/10.3390/polym10101133.
[29] M.S. Lee, J.E. Lee, E. Byun, N.W. Kim, K. Lee, H. Lee, S.J. Sim, D.S. Lee, J.H. Jeong, Target-specific delivery of siRNA by stabilized calcium phosphate nanoparticles using dopa-hyaluronic acid conjugate, J. Control. Release. 192 (2014) 122-130. https://doi.org/10.1016/j.jconre1.2014.06.049.
[30] G. Torrieri, F. Fontana, P. Figueiredo, Z. Liu, M. Ferreira, V. Talman, J.P. Martins, M. Fusciello, K. Moslova, tambet teesalu, V. Ceullo, J. Hirvonen, H. Ruskoaho, V. Balasubramanian, H.A. Santos, Dual-Peptide Functionalized Acetalated Dextran-Based Nanoparticles for Sequential Targeting of Macrophages during Myocardial Infarction, Nanoscale. (2020) 2350-2358. https://doi.org/10.1039/c9nr09934d.
[31] B.L. Bouzo, S. Lores, R. Jatal, S. Alijas, MJ. Alonso, Sphingomyelin nanosystems loaded with uroguanylin and etoposide for treating metastatic colorectal cancer, Sci. Rep. (2021) 1-12. https://doi.org/10.1038/s41598-021-96578-z.
[32] S. Agrawal, M. Dwivedi, H. Ahmad, S.B. Chadchan, A. Arya, R. Sikandar, S. Kaushik, K. Mitra, R.K. Jha, A.K. Dwivedi, CD44 targeting hyaluronic acid coated lapatinib nanocrystals foster the efficacy against triple-negative breast cancer, Nanomedicine Nanotechnology, Biol. Med. 14 (2018) 327-337. https://doi.org/10.1016/j.nano.2017.10.010.
[33] R. Jatal, S. Mendes Saraiva, C. Vázquez-Vázquez, E. Lelievre, O. Coqueret, R. López-López, M. de la Fuente, Sphingomyelin nanosystems decorated with TSP-1 derived peptide targeting senescent cells, Int. J. Pharm. 617 (2022) 121618. https://doi.org/10.1016/j.ijpharm.2022.121618.
[34] S. Díez-Villares, J. Pellico, N. Gómez-Lado, S. Grijalvo, S. Alijas, R. Eritja, F. Herranz, P. Aguiar, M. de la Fuente, Biodistribution of (68/67)Ga-Radiolabeled Sphingolipid Nanoemulsions by PET and SPECT Imaging., Int. J. Nanomedicine. 16 (2021) 5923-5935. https://doi.org/10.2147/IJN.S316767.
[35] M. Mart, S.A. Navarro-marchal, M. Peula-garc, Progress and Hurdles of Therapeutic Nanosystems against Cancer, Pharmaceutics. 14 (2022) 388. https://doi.org/10.3390/pharmaceutics14020388.
[36] AJ. Palmer, H.M. Wallace, The polyamine transport system as a target for anticancer drug development, Amino Acids. 38 (2010) 415-422. https://doi.org/10.1007/s00726-009-0400-2.
[37] S. Nagachinta, G. Becker, S. Dammicco, M.E. Serrano, N. Leroi, M.A. Bahri, A. Plenevaux, C. Lemaire, R. Lopez, A. Luxen, M. de la Fuente, Radiolabelling of lipid-based nanocarriers with fluorine-18 for in vivo tracking by PET, Colloids Surfaces B Biointerfaces. 188 (2020) 110793. https://doi.org/10.1016/j.colsurfb.2020.110793.
[38] S. Mannucci, F. Boschi, B. Cisterna, E. Esposito, R. Cortesi, C. Nastruzzi, E. Cappellozza, P. Bernardi, A. Sbarbati, M. Malatesta, L. Calderan, A correlative imaging study of in vivo and ex vivo biodistribution of solid lipid nanoparticles, Int. J. Nanomedicine. 15 (2020) 1745-1758. https://doi.org/10.2147/IJN.S236968.
[39] AJ. Vázquez-Rios, Á. Molina-Crespo, B.L. Bouzo, R. López-López, G. Moreno-Bueno, M. de la Fuente, Exosome-mimetic nanoplatforms for targeted cancer drug delivery, J. Nanobiotechnology. 17 (2019) 85. https://doi.org/10.1186/s12951-019-0517-8.
[40] S. Wang, M. Shao, Z. Zhong, A. Wang, J. Cao, Y. Lu, Y. Wang, J. Zhang, Co-delivery of gambogic acid and TRAIL plasmid by hyaluronic acid grafted PEI-PLGA nanoparticles for the treatment of triple negative breast cancer, Drug Deliv. 24 (2017) 1791-1800. https://doi.org/10.1080/10717544.2017.1406558.

## Claims

**1.** An isolated polypeptide comprising a fragment of the N-Terminal domain of GSDMB, wherein said fragment is **characterized by** comprising amino acid sequence SEQ ID NO 1 (amino acid sequence 1-233 of the N-Terminal domain of GSDMB) or a functionally equivalent thereof, or a polynucleotide sequence coding for the said polypeptide.

**2.** The polypeptide or polynucleotide sequence according to claim 1, wherein said polypeptide comprises amino acid sequence SEQ ID NO 1 of the N-Terminal domain of GSDB and has a total length of 300 amino acids.

**3.** The polypeptide or polynucleotide sequence according to claim 1, wherein said peptide comprises amino acid sequence SEQ ID NO 1 of the N-Terminal domain of GSDMB and has a total length of less than 275 amino acids.

**4.** The polypeptide or polynucleotide sequence according to claim 1, wherein said peptide consists of amino acid sequence SEQ ID NO 1 of the N-Terminal domain of GSDMB.

**5.** The polynucleotide sequence according to any of claims 1 to 4, wherein the polynucleotide is a DNA sequence, such as a cDNA sequence, or a polyribonucleotide sequence comprising a sequence from a stable mRNA or an mRNA, wherein the DNA sequence or the polyribonucleotide sequence can be efficiently translated to the polypeptide sequence of any of claims 1 to 4.

**6.** The polynucleotide sequence according to any of claims 1 to 5, wherein the polynucleotide is disposed within a vector or plasmid selected for its ability to express the polypeptide in a mammalian cell, preferably in a human cell.

**7.** The vector according to claim 6, wherein the vector is comprised in a nano or micro delivery system comprising vitamin E (vit E) and sphingomyelin (SM) and a cationic or ionizable lipid, preferably a cationic or ionizable lipid comprising putrescine.

**8.** The vector according to claim 7 wherein the vector is comprised in a nanometric or micrometric delivery system comprising vitamin E (vit E), sphingomyelin (SM) and a cationic or ionizable lipid wherein the nanometric or micrometric delivery system is formulated or obtained by an alcohol, such as ethanol, injection method, wherein the vitamin E (VitE), sphingomyelin (SM) and the cationic or ionizable lipid are dissolved in the alcohol and injected in an aqueous medium, with or without additional mixing by homogenization, and wherein the polynucleotide of any of claims 1 to 5, preferably contained in a plasmid, are associated to the said nanometric or micrometric delivery system.

**9.** The nano or microsystem of claim 7 to 8, wherein the nanometric or micrometric delivery system is further **characterized by** being coated with hyaluronic acid or a salt thereof, such as sodium hyaluronate.

**9.** The polypeptide according to any one of claims 1 to 4, the polynucleotide according to any one of claims 1 to 5, or the vector according to any one of claims 6 to 9, for use in therapy.

**11.** The polypeptide according to any one of claims 1 to 4, the polynucleotide according to any one of claims 1 to 5, or the vector according to any one of claims 6 to 9, for use in a method of treatment of a solid tumor, preferably a tumor selected from the following group: breast cancer, pancreatic cancer, lung cancer, prostate cancer, stomach cancer, head and neck cancer, sarcoma, glioblastoma, neuroblastoma, cancer of the colon and rectum, cancer of the head and neck, kidney and bladder cancer, and hepatocarcinoma.

**12.** The polypeptide, polynucleotide, or vector according to claim 11, wherein the use is for treating breast cancer.

**13.** The polypeptide, polynucleotide, or vector according to any of claims 1 to 10, for use in a method of treatment of a solid tumour or cancer metastasis, preferably a solid tumour or cancer metastasis originated from a solid tumor, preferably a tumor selected from the following group: breast cancer, gynecological, pancreas, lung cancer, prostate cancer, stomach cancer, head and neck cancer, sarcoma, glioblastoma, neuroblastoma, cancer of the colon and rectum, cancer of the head and neck, kidney
